# EUROPEAN PATENT APPLICATION

(11) **EP 4 349 941 A1**
(43) Date of publication of application: **10.04.2024**
(21) Application number: 23202531.2
(22) Date of filing: 09.10.2023
(51) Int. Cl.: C10L 3/08, C07C 1/12, C07C 9/04, B01D 53/14, C25B 1/02

(54) **ORGANIC SOLID WASTE TO METHANE FUEL CONVERSION FOR SPACECRAFT**

(30) Priority: 07.10.2022 US 202217961887
(71) Applicant: Hamilton Sundstrand Space Systems International, Inc., Windsor Locks, CT 06096 (US)
(72) Inventor: KHALIL, Yehia F., Glastonbury, CT (US)
(74) Representative: Dehns

(57) **Abstract**

A system includes an oxidative combustion reactor (104) configured to receive solid organic waste and 02, and to output a combined stream of H2O and CO2. A separator (106) is configured to receive the combined stream of H2O and CO2 from the combustion reactor and to separately output a stream of CO2 and a stream of H2O. A Sabatier reactor (108) is operatively connected to receive CO2 from the separator and to receive H2 from an H2 source, and to output gaseous CH4.

## Description

### BACKGROUND

### 1. Field

The present disclosure relates to fuel generation, and more particularly to solid organic waste to methane fuel generation for use in spacecraft propulsion such as for deep space missions such as those for the Artemis Program for deep space exploration and for commercial spacecraft such as the Orbital Reef.

### 2. Description of Related Art

Deep space missions involve distances from Earth to the Moon, Mars, and beyond. Crewed space missions to the Moon take about three days travel time from Earth to the Moon. Space missions to Mars take about seven months travel time from Earth to Mars. Due to long travel times, supplies of fuel to propel and maneuver spacecraft is needed for support of deep space missions.

The conventional techniques have been considered satisfactory for their intended purpose. However, there is an ever-present need for improved systems and methods for fuel supplies for deep space missions and the like. This disclosure provides a solution for this need.

### SUMMARY

A system includes a combustion reactor configured to receive solid organic waste and O₂, and to output a combined stream of H₂O and CO₂. A separator is configured to receive the combined stream of H₂O and CO₂ from the combustion reactor and to separately output a stream of CO₂ and a stream of H₂O. A Sabatier reactor is operatively connected to receive CO₂ from the separator and to receive H₂ from an H₂ source, and to output CH₄.

A mixer can be operatively connected to receive a portion of the O₂ diverted from a supply line supplying the O₂ to the combustion reactor, and to receive CO₂ scrubbed air, and to output a mixture of the diverted O₂ and the CO₂ scrubbed air.

A water electrolyzer can be operatively connected to receive H₂O from the separator through a water supply line, to output the H₂ to the Sabatier Reactor through an H₂ supply line, and to output the O₂ to the supply line supplying the O₂ to the combustion reactor and to the mixer, for oxidizing the solid organic waste with pure O₂ in the combustion reactor. A thermal amine scrubber (TAS) can be configured to receive cabin air that includes CO₂ and to output the CO₂ scrubbed air to the mixer in a scrubbed air line.

A CO₂ accumulator can be connected to a first CO₂ line to receive CO₂ from the TAS. The CO₂ accumulator can be connected to a second CO₂ line to receive CO₂ from the separator. The CO₂ accumulator can be connected to a third CO₂ line to supply CO₂ from the CO₂ accumulator to the Sabatier reactor.

A spacecraft can include a thruster operatively connected to receive CH₄ from the Sabatier reactor for combustion to generate thrust. The spacecraft can include a cabin configured to receive O₂ enriched air from the mixer and to supply CO₂ to the TAS for scrubbing.

A method of producing fuel includes combining CO₂ from a spacecraft cabin and from solid organic waste, and supplying the CO₂ to a Sabatier reactor to produce CH₄ fuel. The method includes generating H₂ and supplying it to the Sabatier reactor for use in generating the CH₄ fuel, wherein generating H₂ includes producing O₂, at least some of which is supplied to the spacecraft cabin for life support.

The method can include mixing CO₂ scrubbed air with the O₂ which is supplied to the cabin for life support. The CO₂ from solid organic waste can be produced by reacting the organic waste with O₂. Reacting the organic waste with O₂ can include combusting the organic waste and O₂ into combustion products including CO₂ and H₂O.

The method can include separating CO₂ from the combustion products for use in the Sabatier Reactor and separating H₂O from the combustion products. The method can include electrolyzing the H₂O from the combustion products into O₂ and H₂ and supplying the H₂ to the Sabatier reactor. The method can include recycling H₂O from the Sabatier reactor to be electrolyzed. The method can include scrubbing CO₂ from the spacecraft cabin for use in the Sabatier reactor. The method can include combusting the CH₄ in a thruster of the spacecraft.

These and other features of the systems and methods of the subject disclosure will become more readily apparent to those skilled in the art from the following detailed description of the preferred embodiments taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, preferred embodiments thereof will be described in detail herein below with reference to certain figures, wherein:

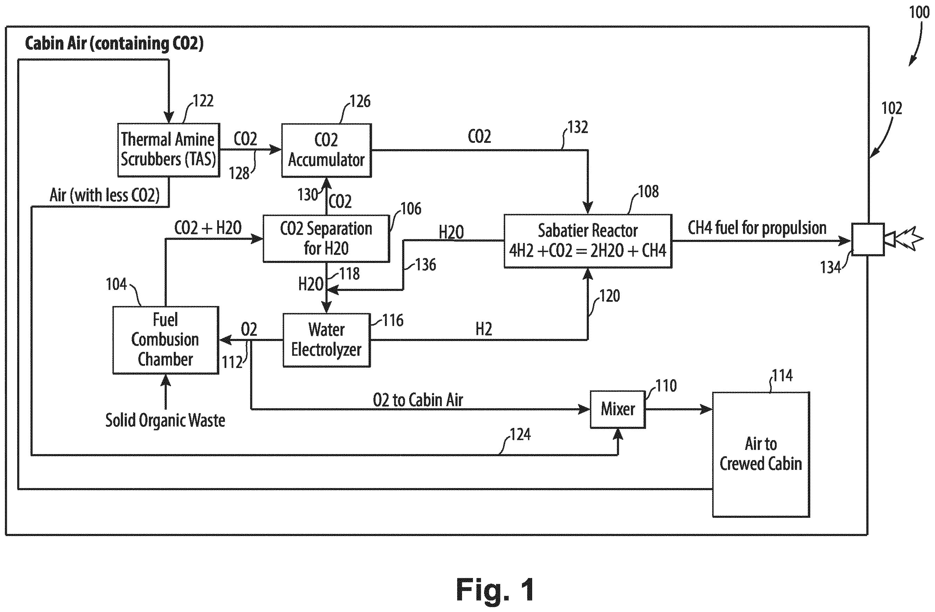

Fig. 1 is a schematic view of an embodiment of a system constructed in accordance with the present disclosure, showing system components used to produce fuel from solid organic waste.

### DETAILED DESCRIPTION OF THE PREFERRED EMBODIMENTS

Reference will now be made to the drawings wherein like reference numerals identify similar structural features or aspects of the subject disclosure. For purposes of explanation and illustration, and not limitation, a partial view of an embodiment of a system in accordance with the disclosure is shown in Fig. 1 and is designated generally by reference character 100. The systems and methods described herein can be used to produce gaseous fuel from solid organic waste, such as leftover food from the crew members and other sources of solid organic matter, for use to propel the spacecraft during deep space missions.

The system 100 can include or be incorporated in a crewed spacecraft 102. The system includes an oxidative combustion reactor 104 configured to receive solid organic waste and O₂, and to output a combined stream of H₂O and CO₂, as indicated by the arrows pointing into and out of the reactor 104 in Fig. 1. A separator 106 is configured to receive the combined stream of H₂O and CO₂ from the combustion reactor 104 and to separately output a stream of CO₂ and a stream of H₂O, as indicated by the arrows into and out of the separator 106 in Fig. 1. A Sabatier reactor 108 is operatively connected to receive CO₂ from the separator 106 and to receive H₂ from an H₂ source, e.g. the water electrolyzer described below, and to output gaseous CH₄, as indicated by the arrows into and out of the reactor 108 in Fig. 1.

A mixer 110 is operatively connected to receive a portion of the O₂ diverted from a supply line 112 supplying the O₂ to the combustion reactor 104, and to receive metabolic CO₂ scrubbed air, e.g. from the thermal amine scrubbers (TAS) described below, and to output a mixture of the diverted O₂ and the metabolic CO₂ scrubbed air, e.g. to the crewed cabin 114 for breathing air for the spacecraft crew members.

A water electrolyzer 116 is operatively connected to receive H₂O from the separator 106 through a water supply line 118, to output the H₂ gas to the Sabatier Reactor 108 through an H₂ supply line 120, and to output the O₂ to the supply line 112 supplying the O₂ to the oxidative combustion reactor 104 and to the mixer 110, e.g., for oxidizing the solid organic waste with pure O₂ in the oxidative combustion reactor. A thermal amine scrubber (TAS) 122 is configured to receive cabin air that includes metabolic CO₂ and to output the CO₂ scrubbed air to the O₂/air mixer 110 in a scrubbed air line 124.

A CO₂ accumulator 126 is connected to a first CO₂ line 128 to receive CO₂ from the TAS 122. The CO₂ accumulator 126 is connected to a second CO₂ line 130 to receive CO₂ from the separator 106. The CO₂ accumulator 126 is connected to a third CO₂ line 132 to supply CO₂ from the CO₂ accumulator 126 to the Sabatier reactor 108.

The spacecraft 102 includes a thruster 134 operatively connected to receive CH₄ from the Sabatier reactor 108, as indicated by the arrow out of the Sabatier reactor 108 in Fig. 1, for combustion to generate thrust. The thruster 134 can be a propulsion thruster, a maneuvering jet, or the like. The spacecraft 102 includes a cabin 114 configured to receive O₂ enriched air from the O₂/air mixer 110 and to supply CO₂ to the TAS 122 for scrubbing.

A method of producing fuel includes combining CO₂ from a spacecraft crew cabin, e.g. cabin 114, and from solid organic waste, and supplying the CO₂ to a Sabatier reactor, e.g. Sabatier reactor 108, to produce CH₄ fuel. The method includes generating H₂ and supplying it to the Sabatier reactor for use in generating the gaseous CH₄ fuel, wherein generating H₂ includes producing O₂, at least some of which is supplied to the spacecraft crew cabin for life support.

The method includes mixing metabolic CO₂ scrubbed air with the O₂ which is supplied to the cabin for life support. The CO₂ from solid organic waste oxidative combustion is produced by reacting the organic waste with O₂, e.g. in the reactor 104. Reacting the organic waste with O₂ includes combusting the organic waste and O₂ into combustion products including gaseous CO₂ and water vapor H₂O.

The method includes separating CO₂ from the oxidative combustion products for use in the Sabatier Reactor and separating H₂O from the combustion products, e.g., in a separator 106. The method includes electrolyzing the H₂O from the combustion products into O₂ and H₂ and supplying the H₂ to the Sabatier reactor, e.g. using an electrolyzer 116. The method includes recycling H₂O from the Sabatier reactor to be electrolyzed, e.g. using the water recycle line 136 feeding into line 118. The method includes scrubbing CO₂ from the spacecraft crew cabin for use in the Sabatier reactor, e.g. using the TAS 122. The method includes combusting the CH₄ in a thruster, e.g., thruster 134, of the spacecraft. Some of the O₂ generated from the water electrolyzer 116 can be used to combust CH₄ in the thruster to propel the spacecraft 102.

The methods and systems of the present disclosure, as described above and shown in the drawings, provide for production of fuel from solid organic waste such as for use during deep space missions. While the apparatus and methods of the subject disclosure have been shown and described with reference to preferred embodiments, those skilled in the art will readily appreciate that changes and/or modifications may be made thereto without departing from the scope of the subject disclosure.

## Claims

1. A system comprising:
a combustion reactor (104) configured to receive solid organic waste and O₂, and to output a combined stream of H₂O and CO₂;
a separator (106) configured to receive the combined gaseous stream of H₂O and CO₂ from the oxidative combustion reactor and to separately output a stream of CO₂ and a stream of H₂O; and
a Sabatier reactor (108) operatively connected to receive CO₂ from the separator and to receive H₂ from an H₂ source, and to output CH₄.

2. The system as recited in claim 1, further comprising:
an O₂/air mixer (110) operatively connected to receive a portion of the O₂ diverted from a supply line (112) supplying the O₂ to the oxidative combustion reactor (104), and to receive metabolic CO₂ scrubbed air, and to output a mixture of the diverted O₂ and the metabolic CO₂ scrubbed air.

3. The system as recited in claim 1 or 2, further comprising a water electrolyzer (116) operatively connected to
receive H₂O from the separator (106) through a water supply line (118);
output the H₂ to the Sabatier Reactor (108) through an H₂ supply line (120); and
output the O₂ to the supply line (112) supplying the O₂ to the combustion reactor and to the mixer (110), for oxidizing the solid organic waste with pure O₂ in the combustion reactor (104).

4. The system as recited in claim 1, 2 or 3, further comprising:
a thermal amine scrubber, TAS, (122) configured to receive cabin air that includes metabolic CO₂ and to output the metabolic CO₂ scrubbed air to the mixer (110) in a scrubbed air line (124).

5. The system as recited in claim 4, further comprising:
a CO₂ accumulator (126) connected to a first CO₂ line (128) to receive CO₂ from the TAS (122), wherein the CO₂ accumulator is connected to a second CO₂ line (130) to receive CO₂ from the separator (106), and wherein the CO₂ accumulator is connected to a third CO₂ line (132) to supply CO₂ from the CO₂ accumulator to the Sabatier reactor (108).

6. The system as recited in claim 4 or 5, further comprising:
a spacecraft (102) including:
a thruster (134) operatively connected to receive CH₄ from the Sabatier reactor (108) for combustion to generate thrust to propel the spacecraft; and
a cabin (114) configured to receive O₂ enriched air from the mixer (110) and to supply CO₂ to the TAS (122) for scrubbing.

7. A method of producing fuel comprising:
combining the metabolic CO₂ from a spacecraft crew cabin and from solid organic waste, and supplying the CO₂ to a Sabatier reactor to produce gaseous CH₄ fuel; and
generating H₂ and supplying it to the Sabatier reactor for use in generating the gaseous CH₄ fuel, wherein generating H₂ includes producing O₂, at least some of which is supplied to the spacecraft crew cabin for life support.

8. The method as recited in claim 7, further comprising mixing metabolic CO₂ scrubbed air with the O₂ which is supplied to the crew cabin for life support.

9. The method as recited in claim 7 or 8, wherein the CO₂ from solid organic waste is produced by reacting the solid organic waste with O₂.

10. The method as recited in claim 9, wherein reacting the solid organic waste with O₂ includes combusting the organic waste and O₂ into combustion products including CO₂ and H₂O.

11. The method as recited in claim 10, further comprising separating CO₂ from the combustion products for use as a feedstock in the Sabatier Reactor and separating H₂O from the combustion products.

12. The method as recited in claim 11, further comprising electrolyzing the H₂O from the combustion products into O₂ and H₂ and supplying the H₂ as a feedstock to the Sabatier reactor.

13. The method as recited in claim 12, further comprising recycling H₂O from the Sabatier reactor to be electrolyzed.

14. The method as recited in any of claims 7 to 13, further comprising scrubbing metabolic CO₂ from the spacecraft crew cabin for use in the Sabatier reactor.

15. The method as recited in claim 14, further comprising combusting the gaseous CH₄ in a thruster of the spacecraft.
